# EUROPEAN PATENT SPECIFICATION

(11) **EP 1 297 174 B1**
(45) Date of publication and mention of the grant of the patent: **17.01.2007**
(21) Application number: 01938473.4
(22) Date of filing: 19.06.2001
(51) Int. Cl.: C12Q 1/00

(54) **INTERACTION OF TBX2 AND THE ARF PROMOTER IN CELL CYCLE REGULATOR ASSAYS**
WECHSELWIRKUNG ZWISCHEN TBX2 UND DEM ARF-PROMOTER IN ZELLZYKLUS-REGULATOR-ASSAYS
INTERACTION ENTRE TBX2 ET LE PROMOTEUR D'ARF DANS DES TESTS AYANT POUR SUJET DES REGULATEURS DU CYCLE CELLULAIRE

(30) Priority: 19.06.2000 US 212627 P
(43) Date of publication of application: 02.04.2003
(73) Proprietor: Vereniging Het Nederlands Kanker Instituut, Nl-1066 CX Amsterdam (NL)
(72) Inventor: VAN LOHUIZEN, Maarten Div. Mole. Carcinogenesis, NL-1066 CX Amsterdam (NL); JACOBS, Jacqueline Div. Molecular Carcinogenesis, NL-1066 CX Amsterdam (NL); VAN DE VIJVER, Marc, NL-1066 CX Amsterdam (NL)
(74) Representative: Brasnett, Adrian Hugh
(86) International application number: PCT/GB2001/002706
(87) International publication number: WO 2001/098530

(56) References cited:
- WO-A-01/55450
- ZINDY FREDERIQUE ET AL: "Myc signaling via the ARF tumor suppressor regulates p53-dependent apoptosis and immortalization." GENES & DEVELOPMENT, vol. 12, no. 15, 1 August 1998 (1998-08-01), pages 2424-2433, XP001098432 ISSN: 0890-9369
- PAPAIOANNOU VIRGINIA E ET AL: "The T-box gene family." BIOESSAYS, vol. 20, no. 1, January 1998 (1998-01), pages 9-19, XP002210227 ISSN: 0265-9247
- CHEN J ET AL: "Microarray analysis of Tbx2-directed gene expression: a possible role in osteogenesis." MOLECULAR AND CELLULAR ENDOCRINOLOGY. IRELAND 25 MAY 2001, vol. 177, no. 1-2, 25 May 2001 (2001-05-25), pages 43-54, XP001098430 ISSN: 0303-7207
- LINGBEEK MEREL E ET AL: "The T-box Repressors TBX2 and TBX3 Specifically Regulate the Tumor Suppressor Gene p14ARF via a Variant T-site in the Initiator." JOURNAL OF BIOLOGICAL CHEMISTRY, (2002 JUL 19) 277 (29) 26120-7. , XP001098435

## Description

### Field of the Invention.

The present invention relates to the TBX2 gene and its use in assays for inhibitors of cell proliferation. To identify novel immortalizing genes with potential roles in tumourigenesis, we performed a genetic screen, aimed to bypass the rapid and tight senescence arrest of primary fibroblasts deficient for the *Bmi1* oncogene. We identified the T-box member *TBX2* as a potent immortalizing gene that acts by down-regulating p19^{ARF}. TBX2 represses the *ARF* promoter and attenuates E2F1, Myc or Ras-mediated induction of p19^{ARF}. Interestingly, we found *TBX2* to be amplified in a subset of primary human breast cancers, suggesting that it might contribute to breast cancer development.

### Background to the Invention.

Due to the existence of several control mechanisms, the lifespan of normal mammalian cells is limited, ending either in an irreversible growth arrest called replicative senescence, or in apoptosis. In cancer cells, these control mechanisms are disabled leading to continued and uncontrolled proliferation¹. One important control mechanism depends on the tumor suppressor p53 (ref. 2), whose inactivation is the most frequent event in human cancer. P53 is a transcription factor which blocks cell cycle progression or induces apoptosis in response to DNA damage or other types of stress. A second control mechanism that prevents cells from indefinite proliferation is governed by the CDK inhibitor p16^{INK4a} (ref. 3), whose levels rise with accumulating population doublings (refs. 4-6). Binding of p16^{INK4a} to CDK4 and 6 (ref. 3) induces a G1 phase cell cycle arrest by preventing the inactivation of the tumor suppressor RB. p16^{INK4a} is encoded by the *INK4a*/*ARF* tumour suppressor locus (refs. 7, 8). Next to the RB pathway, *INK4a*/*ARF* also controls the p53 pathway by generating an alternative transcript that encodes for p19^{ARF} (refs 9-13). p19^{ARF} sequesters the oncoprotein MDM2 to the nucleolus (ref. 14) and blocks nucleo-cytoplasmic shuttling of MDM2 (ref.15). This prevents p53 degradation by MDM2 and leads to increased p53 stability and function(ref. 16). Given its unusual ability to regulate both the RB and p53 tumor suppressor pathways it is not surprising that disruption of the *INK4a*/*ARF* locus is common to many cancers (ref. 5).

*INK4a*/*ARF* not only functions to halt cell proliferation upon a maximum number of cell divisions but also upon oncogenic, hyper-proliferative signalling (refs. 7, 8). In primary cells, E1A, oncogenic Ras or Raf, Myc, Abelson and E2F elicit an apoptotic response or a senescence-like growth arrest by inducing p19^{ARF} and/or p16^{INK4a} expression (refs. 7, 8).
Efficient oncogenic transformation by these factors is only achieved when p53, RB or INK4a/ARF function is compromised, explaining for instance the strong selection pressure for disruption of the INK4a/ARF-MDM2-p53 pathway during Myc-induced lymphomagenesis (refs. 17-19).

Previously, we identified the *INK4a*/*ARF* locus as a critical target of the Polycomb-group repressor Bmi1 in its ability to regulate cell proliferation and immortalization (ref. 20). Over-expression of Bmi1 in primary mouse embryo fibroblasts (MEFs) results in down-regulation of p16^{INK4a} and p19^{ARF}, causing extension of cellular lifespan, increased proliferation and neoplastic transformation in cooperation with oncogenic Ras or Myc. *In vivo,* Bmi1 over-expression predisposes mice to lymphomagenesis, which is severely enhanced upon co-over-expression of Myc (refs. 21, 22). This collaboration is based on Bmi1's ability to inhibit the apoptotic response elicited by Myc, by preventing Myc-mediated p19^{ARF} induction (ref. 19). Conversely, absence of Bmi1 causes de-repression of p16^{INK4a} and p19^{ARF}, leading to premature senescence of MEFs and severe proliferation defects in lymphoid organs and cerebellum, which is largely rescued upon disruption of the *INK4a*/*ARF* locus (ref. 20).

### Disclosure of the Invention.

Genes, whose products negatively regulate the INK4a/ARF pathway are expected to act as immortalizing oncogenes and may contribute to tumourigenesis. To identify such novel regulators, we screened a retroviral cDNA library for genes capable of immortalizing *Bmi1-*/*-* MEFs. By using these senescence-predisposed MEFs, the screen was expected to select especially for strong inhibitors of the INK4a/ARF pathway and to be accompanied by a low background of false positives due to spontaneous immortalization. In addition, apart from their de-repression in *Bmil-*/*-* MEFs, the p16^{INK4a} and p19^{ARF} genes remain under the control of normal regulatory elements in the *INK4a*/*ARF* locus. This permits the screen to identify relevant direct upstream regulators of *INK4a*/*ARF*.

The present invention relates to the identification of *TBX2* by this screen, as a gene that immortalizes MEFs by down-regulating primarily p19^{ARF}. In addition, we have found *TBX2* to be amplified in a subset of primary human breast carcinomas, suggesting a potential role for TBX2 in the development of these tumors.

In summary, we have found that the T-box transcription factor TBX2 binds to the ARF promoter, and that this binding results in repression of this promoter. The promoter repression prevents the induction of p19^{ARF} upon oncogenic hyperproliferative signals, leading to progression of cells through the cell cycle.

Although TBX2 has previously been found to exhibit DNA binding activity, the consensus binding site does not appear to be involved in the novel repression that we have identified. Instead, TBX2 interacts with a region of the ARF promoter through an element 3' of position -19 of the promoter. Furthermore, deletion of the repression domain of TBX2 severely reduces its ability to repress the human ARF promoter.

Thus in a first aspect, the invention provides an assay for a modulator of cell cycle progression, which comprises:
a) bringing into contact a TBX2 protein, an ARF promoter TBX-2 binding region and a putative inhibitor compound under conditions where the TBX2 protein and the binding region, in the absence of inhibitor, are capable of forming a complex; and
b) measuring the degree of inhibition of binding or transcriptional activation caused by said inhibitor compound.

We have also found that the TBX2 gene is in a region of the chromosome which is often amplified in breast tumour cells. Thus in another aspect, the invention,provides a diagnostic or prognostic method for the treatment or prognosis of breast cancer, the method comprising determining the level of TBX2 nucleic acid in a cell which is, or is suspected to be, a breast tumour cell or metastatic cell from a breast tumour. The determining may be of TBX2 copy number or the level of expression.

### Brief Description of the Drawings.

Figures 1a-c shows TBX2 immortalizes MEFs and delays senescence of primary human fibroblasts.
Figures 2a-c shows TBX2 down-regulates INK4a/ARF.
Figures 3a-c shows TBX2 leaves pathways downstream of INK4a/ARF intact.
Figures 4a & b shows TBX2 counteracts induction of p19^{ARF} by Myc and Ras.
Figures 5a-e shows TBX2 represses the ARF promoter.

### Detailed Description of the Invention.

The TBX2 may be any suitable mammalian TBX2, particularly human TBX2. Human TBX2 is a 701 amino acid protein whose gene has been cloned, so that sources of the gene can be readily identified by those of skill in the art. Its sequence may be found by reference to Genbank (entry S81264) or Swiss-Prot (entry Q13207). Murine TBX2 has also been cloned (Swiss Prot Q60707) and is 94% similar over its 701 amino acids to the human protein sequence. Other mammalian TBX2 genes can be obtained using routine cloning methods and/or by reference to databases. For example, a rat TBX2 protein (PID:g2760445) has been identified, as well as non-mammalian homologues *(D.melanogaster* Swiss-Prot Q24432, *C.elegans* PID: g506796).

Although a wild-type TBX2 is preferred (particularly a human or murine wild-type), variants of TBX2 which still retain the ability to interact directly with the ARF binding site may also be used, such as natural or synthetic variants having at least 70%, preferably at least 80%, more preferably at least 90%, even more preferably at least 95% and most preferably at least 99% identity to the human TBX2 protein.

The percentage identity of amino acid sequences can be calculated using commercially available algorithms. The following programs (provided by the National Center for Biotechnology Information) may be used to determine homologies: BLAST, gapped BLAST, BLASTN and PSI-BLAST, which may be used with default parameters.

The algorithm GAP (Genetics Computer Group, Madison, WI). GAP uses the Needleman and Wunsch algorithm to align two complete sequences that maximizes the number of matches and minimizes the number of gaps. Generally, the default parameters are used, with a gap creation penalty = 12 and gap extension penalty = 4.

It is not necessary to use the entire TBX2 protein for assays of the invention. Fragments of the protein may be used provided such fragments retain the ability to interact with the target domain of the ARF TBX2-binding region responsible for the TBX2 interaction. Fragments of TBX2 may be generated in any suitable way known to those of skill in the art. Suitable ways include, but are not limited to, recombinant expression of a fragment of the DNA encoding the TBX2. Such fragments may be generated by taking DNA encoding the protein, identifying suitable restriction enzyme recognition sites either side of the portion to be expressed, and cutting out said portion from the DNA. The portion may then be operably linked to a suitable promoter in a standard commercially available expression system. Another recombinant approach is to amplify the relevant portion of the DNA with suitable PCR primers. These types of approaches may be used to generate fragments of, for example 100-700, e.g 200-700, such as 300-700, 400-700, 500-700 or 600-700 amino acids of the TBX2 protein.

Small fragments of the protein (up to about 20 or 30 amino acids) may also be generated using peptide synthesis methods which are well known in the art.

The ability of suitable fragments to bind to the ARF TBX2-binding region (or fragment thereof) may be tested using routine procedures such as those described in the accompanying examples. Reference herein to TBX2 protein includes the above mentioned variants and fragments which are functionally able to bind the ARF TBX2-binding region unless the context is explicitly to the contrary.

The ARF TBX2-binding region used in the assay may be obtained from the same mammalian source as the TBX2 protein. The ARF TBX2-binding region is preferred.

The ARF TBX2-binding region is located in the ARF promoter downstream of -19. The region from -19 to +54 (with the initiator sequence underlined) is shown as SEQ ID NO:1:

TC TGCAGTTAAG GGGGCAGGAG TGGCGCTGCT CACCTCTGGT GCCAAAGGGC
GGCGCAGCGG CTGCCGAGCT C (SEQ ID NO:1)

We have found that this sequence, when used in a bandshift assay in the presence of cell components which include TBX2, results in a TBX2-containing complex which is super-shifted by antibodies against TBX2.

Thus this DNA region, or fragments thereof which retain TBX2 binding activity may be used in assays of the invention. Of course, those of skill in the art will appreciate that this region may be used in the form of a larger ARF promoter and/or transcribed region, or may be linked to an alternative transcribed region, such as a reporter gene. Specific fragments include SEQ ID NO:6 and SEQ ID NO:4 identified herein below.

Those of skill in the art will also appreciate that the TBX2-binding region is comprised of specific nucleotides in the -19 to +54 region which contact the protein. These nucleotides are determined by, for example, DNA footprinting studies known per se to those of skill in the art. Such techniques are used to indicate the essential nucleotides for TBX2 binding and are retained in the assays of the invention and in reporter gene constructs. Nucleotides other than those which make the essential contacts may be substituted by any other nucleotide. Nucleotides within a T-box-like region of SEQ ID NO:1 which are conserved in the consensus T-box region or found by X-ray crystallography analysis to make important contacts with the T-box are indicated below in the accompanying examples.

The interaction between TBX2 protein and the ARF TBX2-binding region may be studied *in vitro* by labelling one of these components with a detectable label and bringing it into contact with the other component which has been optionally immobilised on a solid support, either prior to or after the components have been brought into contact with each other. Suitable detectable labels include ³⁵S-methionine which may be incorporated into recombinantly produced proteins, and tags such as an HA tag, GST, MBP (Maltose Binding Protein) or histidine. The recombinantly produced protein may also be expressed as a fusion protein containing an epitope which can be labelled with an antibody. Alternatively, an antibody against the TBX2 can be obtained using conventional methodology. Likewise, the binding region may be labelled, for example with ³²P or biotin.

The component which is optionally immobilized on a solid support may be immobilized using technologies which are known *per se,* for example in the case of TBX2 by an antibody. A fusion protein of TBX2 fused to glutathione-S-transferase (GST) provides an alternative means. This may be immobilized on glutathione agarose beads. In an *in vitro* assay format of the type described above the putative inhibitor compound can be assayed for example by determining its ability to diminish the amount of labelled TBX2 which binds to the nucleic acid comprising the binding region bound to a solid support. The solid support may be rinsed to remove unbound protein and the amount of protein which has bound can be determined by counting the amount of label present in, for example, a suitable scintillation counter.

In an alternative mode, the one of the TBX2 and the binding region may be labelled with a fluorescent donor moiety and the other labelled with an acceptor which is capable of reducing the emission from the donor. This allows an assay according to the invention to be conducted by fluorescence resonance energy transfer (FRET). In this mode, the fluorescence signal of the donor will be altered when the two components interact. The presence of a candidate inhibitor compound which disrupts the interaction will increase the amount of unaltered fluorescence signal of the donor.

FRET is a technique known per se in the art and thus the precise donor and acceptor molecules and the means by which they are linked to the two components may be accomplished by reference to the literature.

Suitable fluorescent donor moieties are those capable of transferring fluorogenic energy to another fluorogenic molecule or part of a compound and include, but are not limited to, coumarins and related dyes such as fluoresceins, rhodols and rhodamines, resorufins, cyanine dyes, bimanes, acridines, isoindoles, dansyl dyes, aminophthalic hydrazines such as luminol and isoluminol derivatives, aminophthalimides, aminonaphthalimides, aminobenzofurans, aminoquinolines, dicyanohydroquinones, and europium and terbium complexes and related compounds.

Suitable acceptors include, but are not limited to, coumarins and related fluorophores, xanthenes such as fluoresceins, rhodols and rhodamines, resorufins, cyanines, difluoroboradiazaindacenes, and phthalocyanines.

A preferred donor is fluorescein and preferred acceptors include rhodamine and carbocyanine. The isothiocyanate derivatives of these fluorescein and rhodamine, available from Aldrich Chemical Company Ltd, Gillingham, Dorset, UK, may be used to label the TBX2 and the binding region. For attachment of carbocyanine, see for example Guo et al, J. Biol. Chem., **270**; 27562-8, 1995.

The assay of the invention may also take the form of an *in vivo* assay. The *in vivo* assay may be performed in a cell line in which the ARF TBX2-binding region is operably linked to a reporter gene. Inhibition of the interaction will provide for increased levels of expression of the reporter gene. Two or more binding regions (for example 3, 4 or 5) may be present in the construct and this may enhance sensitivity of the assay. The reporter gene may be any suitable reporter gene used in the art. Such reporter genes includes chloramphenicol acetyl transferase (CAT), luciferase, green fluorescent protein and the like.

In a further aspect, the finding that transcriptional activation of ARF by oncogenes, particularly Myc, Ras, E1A and E2F-1, is effectively blocked by over-expression of TBX2 provides for an assay in which the induction of ARF by these components is determined when these components are activated.

The cell lines used in assays of the invention may be used to achieve transient expression of the reporter although in a further aspect of the invention cells which are stably transfected with constructs which express the reporter may also be generated. Means to generated stably transformed cell lines are well known in the art and such means may be used here.

Suitable cells include mammalian (e.g. COS7), yeast, insect or bacterial cells. Where the cell line does not express TBX2, a construct capable of expressing this protein may also be introduced into the cell operably linked to a suitable promoter.
The precise format of the assays of the invention may be varied by those of skill in the art using routine skill and knowledge. In the *in vitro* assays of the invention, the amount of TBX2 and binding region may be varied depending upon the scale of the assay. In general, the person of skill in the art will select relatively equimolar amounts of the two components, say from 1:10 to 1:100, preferably from 1:1 to 1:10, molar ratio of the two components. However there may be particular assay formats which can be practised outside this range.

The amount of putative inhibitor compound which may be added to an assay of the invention will normally be determined by trial and error depending upon the type of compound used. Typically, from about 0.01 to 100 µM concentrations of putative inhibitor compound may be used, for example from 0.1 to 10 µM.

Inhibitor compounds which may be used may be natural or synthetic chemical compounds used in drug screening programmes. For example, peptide aptamer libraries may be used. Extracts of plants which contain several characterised or uncharacterised components may also be used. A further class of putative inhibitor compounds can be derived from the TBX2 protein sequence. Peptide fragments of from 5 to 40 amino acids, for example from 6 to 10 amino acids from the region of TBX2 which is responsible for the interaction between it and the binding region may be tested for their ability to disrupt this interaction. Antibodies directed to the site of interaction in TBX2 form a further class of putative inhibitor compounds. Candidate inhibitor antibodies may be characterised and their binding regions determined to provide single chain antibodies and fragments thereof which are responsible for disrupting the interaction between TBX2 and the binding region.

We have also found that a small oligonucleotide sequence is capable of competing out the binding of TBX2 to the ARF promoter region, and thus nucleotides based upon this sequence (SEQ ID NO:4) may also be used and investigated in assays of the invention. Synthetic oligonucleotides such as those with modified backbone structures may be used in this aspect of the invention.

Other candidate inhibitor compounds may be based on modelling the 3-dimensional structure of TBX2 and using rational drug design to provide potential inhibitor compounds with particular molecular shape, size and charge characteristics.

Candidate inhibitor compounds obtained according to the method of the invention may be prepared as a pharmaceutical preparation. Such preparations will comprise the compound together with suitable carriers, diluents and excipients. Such formulations form a further aspect of the present invention. The formulations may be used in methods of treatment of proliferative diseases, for example, breast cancer.

Candidate inhibitor compounds may also be used in combination with any other anti-proliferative compounds used to treat hyperproliferative diseases such as cancers. In such a case, the assay of the invention, when conducted *in vivo*, need not measure the degree of inhibition of binding or transcriptional activation caused by the inhibitor compound being tested. Instead the effect on cell growth or proliferation may be measured. It may be that such a modified assay is run in parallel or subsequent to the main assay of the invention in order to confirm that any effect on cell growth or proliferation is as a result of the inhibition of binding or transcriptional activation caused by said inhibitor compound and not merely a general toxic effect.

Breast tumours are often estrogen responsive although other tumour types which have been found to be estrogen-responsive include endometrial cancer and ovarian carcinoma. Thus inhibitor compounds obtained by the present invention may be used in combination with anti-estrogens such as tamoxifens, e.g. 4-hydroxytamoxifen or pure anti-estrogens such as N-(n-butyl)-11-[3,17beta-dihydroxy-estra-1,3,5(10)-trien-7alpha-yl]N-methylundecanamide, known as ICI 164,384. Such anti-estrogen compounds may be included in the assay in order to determine inhibitor compounds which may act additively or synergistically with anti-estrogens. In addition, TBX2 inhibitors may be used in combination with cytotoxic compounds for estrogen-responsive or anti-estrogen resistant tumours.

In the diagnostic and prognostic application of the invention, there is provided a method which involves determining the level of expression and/or amplification of the TBX2 gene.

In particular, the level of amplification of the gene may be determined, for example by determining the copy number of the TBX2 genomic DNA in a cell. This may be done by measuring the amount of TBX2 in relation to an unamplified reference gene, for example using Southern analysis or PCR. Any reference gene outside the chromosome 17q22-24 region may be used.

Conveniently, such a gene may be SRC-1. Means to quantitate the amount of DNA or its relative copy number in a cell are known *per se* in the art. Reference may be made to, for example, Sambrook, Fritsch and Maniatis, "Molecular Cloning, A Laboratory Manual", Cold Spring Harbor Laboratory Press, 1989, and Ausubel et al, Short Protocols in Molecular Biology, John Wiley and Sons, 1992.

Measurement of the TBX2 gene may be achieved by use of a probe directed to a TBX2 exon, intron, 5' or 3' untranslated regions, or the promoter region.

Alternatively, the level of TBX2 expression may be determined, by determination of the amount of TBX2 mRNA or the amount of TBX2 protein. The amount of TBX2 protein may be determined by the use of antibodies against this protein. Such antibodies may be monoclonal or polyclonal, raised using conventional technology. The antibodies may also be obtained from recombinant libraries of natural, synthetic or partially synthetic genes, such as phage display libraries. Antibodies may be complete antibodies or fragments thereof which retain binding to TBX2, such as scFv or Fab fragments. The levels of protein may be determined by competitive or non-competitive immunoassays whose formats are known as such in the art.

The determination of the levels of TBX2 nucleic acid or protein in a sample of breast tumour cells may be used as an indicator of the therapeutic regime to be administered to a patient. For example, high levels of amplification (i.e. levels above a copy number ratio of 1.15, such as above 1.8) may indicate the need for more intense or prolonged chemotherapeutic treatment than for a patient in which there is a low TBX2 copy number (i.e. less than 1.15).

Likewise, the copy number or expression level of TBX2 in breast cells following a course of treatment may be determined in order to gauge the success of the treatment or the need for further treatment.

Moreover, biopsy samples from patients in which breast cancer is suspected may be subject to a determination of TBX2 copy number or expression level as a marker for the presence of the disease, wherein increased copy number is used as a positive indicator for a malignant or pre-malignant state, facilitating a prompt start to treatment or surgery.

The demonstration that TBX2 plays a significant role in repression of the ARF promoter leads to a further aspect of the present invention. Interaction between TBX2 protein and the ARF TBX2-binding region may be inhibited by inhibition of the production of the TBX2 protein. Such treatment may therefore form the basis of therapy to, for example, prevent or treat breast cancer. For instance, production of the TBX2 protein may be inhibited by using appropriate nucleic acid to influence expression by antisense regulation. The use of anti-sense genes or partial gene sequences to down-regulate gene expression is now well-established. Double-stranded DNA is placed under the control of a promoter in a "reverse orientation" such that transcription of the "anti-sense" strand of the DNA yields RNA which is complementary to normal mRNA transcribed from the "sense" strand of the target gene. The complementary anti-sense RNA sequence is thought then to bind with mRNA to form a duplex, inhibiting translation of the endogenous mRNA from the target gene into protein. Whether or not this is the actual mode of action is still uncertain. However, it is established fact that the technique works.

An alternative to anti-sense is to use a copy of all or part of the target gene inserted in sense, that is the same, orientation as the target gene, to achieve reduction in expression of the target gene by co-suppression. See, for example, US-A-5,231,020.

A further possibility is to use double stranded RNA (dsRNA) to silence genes. On introduction of dsRNA into a cell corresponding to a sense and antisense sequence of an endogenous mRNA , degradation of the cognate mRNA occurs and the gene is silenced. This has been shown in cells from a large variety of organisms including plants, nematodes, fruit flies and mice and is reviewed by Fire (1999) Trends in Genetics 15:358-363 and Bass (2000) Cell 101:235-238.

Another possibility is that nucleic acid is used which on transcription produces a ribozyme, able to cut nucleic acid at a specific site - thus also useful in influencing gene expression. Background references for ribozymes include Kashani-Sabet and Scanlon (1995). *Cancer Gene Therapy,* **2**, (3) 213-223, and Mercola and Cohen (1995). *Cancer Gene Therapy* **2**,(1) 47-59.

The following examples illustrate the invention.

### Identification of TBX2

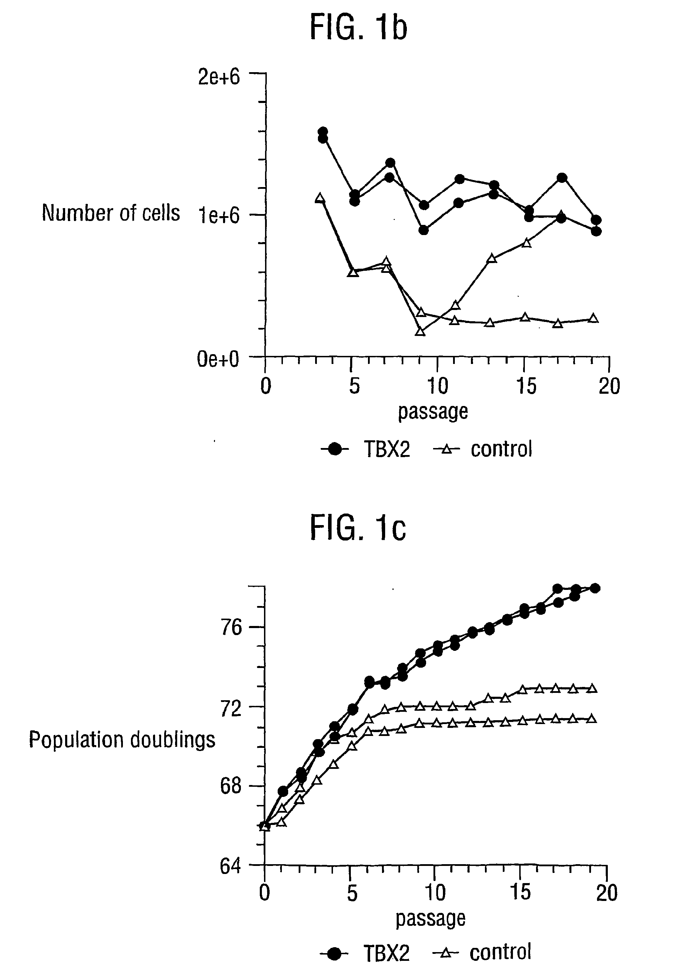

*Bmi1-*/*-* MEFs rapidly accumulate increased, but importantly still physiological relevant levels of p16^{INK4a} and p19^{ARF} which drives such cells within 3 passages into a very tight premature senescence arrest, from which the spontaneous escape rate is low (ref. 20). To identify genes that are able to immortalize these MEFs we infected passage 2 *Bmi1-*/*-* MEFs with a replication deficient retroviral cDNA library derived from the JEG3 chorio-carcinoma cell line. Infected cells were passaged twice, resulting in a completely senescent cell population. After 2 to 3 weeks immortal clones arose from this senescent population, which were isolated and expanded. To distinguish clones rescued by a cDNA insert from those that escaped senescence spontaneously, individual clones were infected with wild-type replication competent MoMuLV as a tool to mobilize retroviral cDNA inserts. The virus produced as a result of the mobilization was used to perform a secondary screen by infecting fresh *Bmi1-*/*-* MEFs followed by analysis for escape from senescence. In each of 5 screens we identified one cDNA multiple times, which was able to efficiently rescue premature senescence and to immortalize *Bmi1-*/*-* MEFs in primary and secondary screens. Interestingly, MEFs expressing this cDNA adopted a morphology that was clearly distinct from that of normal MEFs and Bmi1-immortalized MEFs (Fig. 1b). Southern blot analysis of the rescued cells revealed a 3.9 kb insert that was cloned via a size-selected phage library using a library specific probe. The insert represents the complete cDNA for human *TBX2* (refs 23,24), a member of the T-box family (ref. 25) of transcription factors that is named after the prototype of this family: Brachyury or 'T'. Notably, T-box transcription factors are well known for their involvement in different developmental processes, but have to our knowledge not been associated with regulation of the cell cycle or senescence checkpoint before.

### TBX2 readily immortalizes MEFs and delays senescence of primary human fibroblasts.

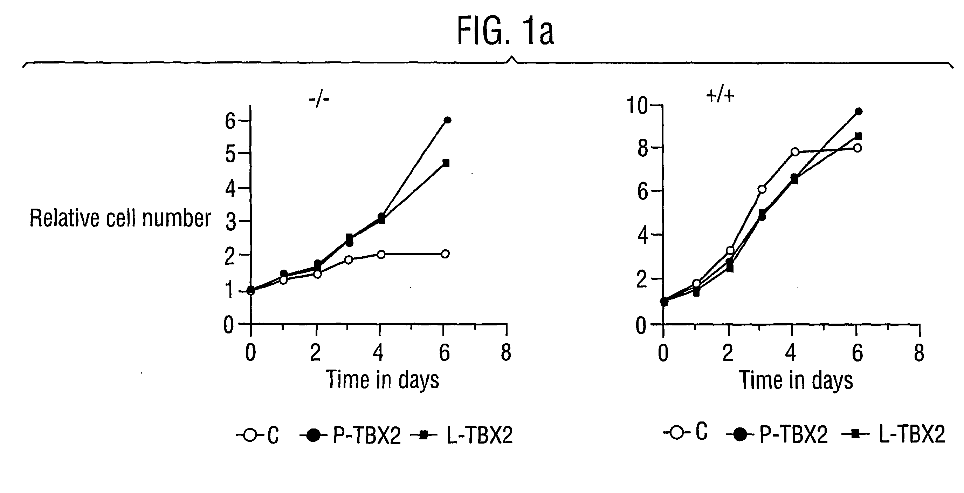

Recloning the *TBX2* cDNA in the PMX retroviral library vector or in the LZRS-iresGFP retrovirus, followed by infection of *Bmil-*/- MEFs with these viruses, confirmed that TBX2 prevents premature senescence and immortalizes *Bmi1-*/*-* MEFs (Fig. 1a). In contrast to Bmi1, which not only delays senescence but also increases the proliferation rate of wild-type MEFs (ref. 20), TBX2 does not confer an additional growth advantage to early passage wild-type MEFs (Fig. 1a). However, at later passages, when wild-type MEFs approach senescence and proliferation slows down, TBX2 over-expressing MEFs continue to proliferate at a constant rate. This is best illustrated when MEFs are serially passaged on a 3T3 schedule. Whereas wild-type MEFs enter senescence after about 7 passages, from which rare cells eventually escape and give rise to an immortal culture, TBX2 over-expressing MEFs continue to proliferate for over 30 passages (Fig. 1b). This suggests that immortalization by TBX2 does not require additional mutational events. Indeed, we found no mutation of p53 or loss of the *INK4a*/*ARF* locus in any of 18 independently propagated TBX2 over-expressing MEF cultures after more than 22 passages: Interestingly, over-expression of TBX2 in TIG3 primary human fibroblasts causes a significant delay in senescence entry (Fig. 1c), suggesting that the mechanism through which TBX2 interferes with the senescence checkpoint is conserved in human cells.

*Detailed legend to Figure 1: **a***, Growth curves of passage 3 *Bmi1-*/*-* and wild-type MEFs infected at passage 1 with empty control (C), PMX(P)-TBX2 or LZRS(L)-TBX2 viruses. Standard deviations were within 10% of the means shown. ***b***, Proliferation of control or TBX2 virus infected MEFs on a 3T3 schedule. ***c***, Proliferation of human TIG3 fibroblasts on a 3T3 schedule, showing extension of life span by TBX2.

### TBX2 down-regulates INK4a/ARF.

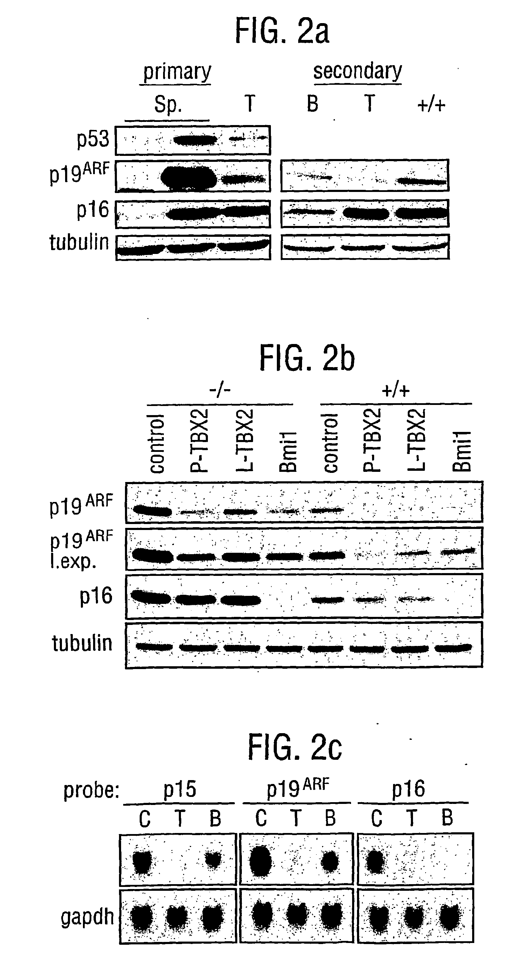

As premature senescence of *Bmi1-*/*-* MEFs is due to rapid accumulation of p16^{INK4a} and p19^{ARF} and can be rescued by down-regulation or abolishment of their expression, we analyzed the p16^{INK4a} and p19^{ARF} levels of *Bmi1-*/*-* primary and secondary rescued colonies. The very low p19^{ARF} levels in the TBX2-rescued clones suggested that TBX2 might act as a negative regulator of p19^{ARF} (Fig. 2a). Whereas in clones rescued from premature senescence by over-expression of Bmi1 both p19^{ARF} and p16^{INK4a} levels are significantly reduced, TBX2-rescued clones contain p16^{INK4a} levels that equal levels found in senescent cells. This suggests that down-regulation of p19^{ARF} is the primary cause of the sustained senescence bypass by TBX2 (Fig. 2a). Indeed, when *Bmi1-*/*-* or wild-type MEFs were infected with TBX2 retroviruses and analyzed within 2 passages after infection, we found a strong down-regulation of p19^{ARF} levels as opposed to a relatively mild reduction in p16^{INK4a} protein levels (Fig. 2b). Northern analysis indicated that both p19^{ARF} and p16^{INK4a} are down-regulated at the transcriptional level (Fig. 2c). The reduction of p16^{INK4a} protein levels by TBX2 appears weaker than expected from the amount of reduction of p16^{INK4a} transcript levels (compare Fig. 2b with 2c). This might be explained by the long half-life of the p16^{INK4a} protein (ref. 6). In addition, TBX2 also down-regulates transcription from the neighboring *INK4b* gene, that encodes the CDK-inhibitor p15^{INK4b} (ref 26). Both Northern and Western analysis clearly indicated that TBX2 and Bmi1 act in different ways: Bmi1 affects mostly p16^{INK4a} and to a lesser extent p19^{ARF}, whereas TBX2 affects predominantly p19^{ARF} and p15^{INK4b} and less so p16^{INK4a} (Fig. 2b,c).

*Detailed legend to Figure* 2: ***a***, Western analysis of p53 and INK4a/ARF status in cells rescued in the primary and secondary screen. Rare clones unable to produce virus that rescues in a secondary screen, represent spontaneous escape from senescence either through loss of expression from the *INK4a*/*ARF* locus, or through mutation and stabilization of p53, the latter resulting in increased p19^{ARF} levels.(Sp: spontaneous immortalized clones; T: TBX2 rescued clone; B: MoMuLV/Bmil rescued cells; T: MoMuLV/TBX2 rescued cells; +/+: senescent wild-type MEFs) ***b***, Western analysis of p16^{INK4a} and p19^{ARF} protein levels (l.exp: longer exposure) in *Bmi1-*/*-* (-/-) and wild-type (+/+) MEFs 2 passages after infection with control, Bmi1 or 2 different TBX2 retroviruses. Tubulin levels serve as loading control. ***c***, Northern analysis of p15^{INK4b}, p19^{ARF} and p16^{INK4a} transcript levels in wild-type MEFs 2 passages after infection with control (C), Bmi1 (B) or TBX2 (T) retroviruses. GAPDH was included as a loading control.

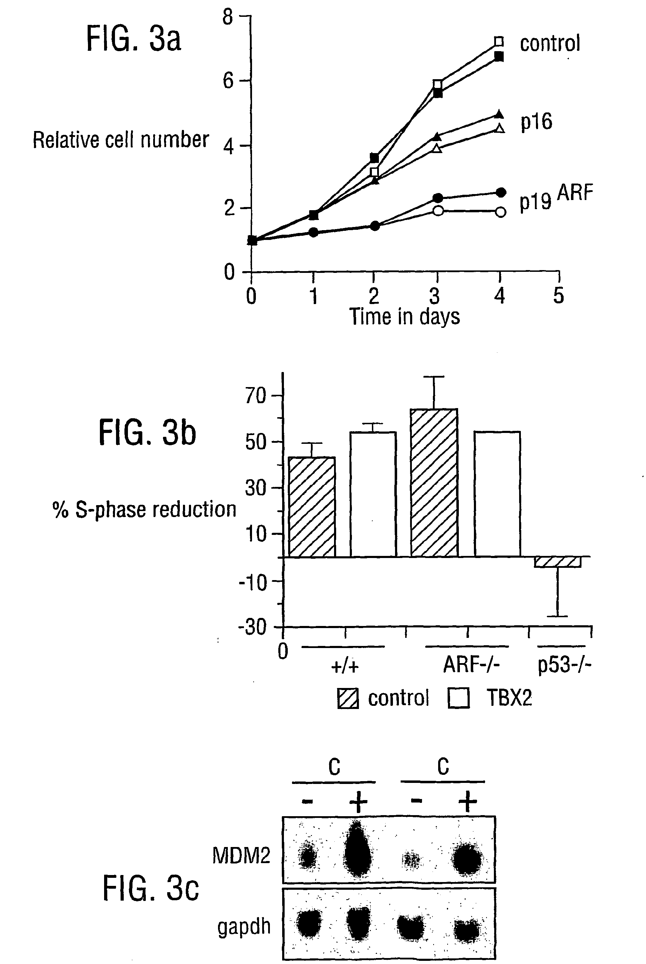

Consistent with TBX2 acting upstream of *INK4a*/*ARF*, over-expression of exogenous p16^{INK4a} or p19^{ARF} could still inhibit proliferation of TBX2 over-expressing MEFs, indicating that TBX2 does not inactivate major downstream effectors of *INK4a*/*ARF* (Fig. 3a). Although p19^{ARF} controls proliferation and senescence entry by modulating p53 activity, p19^{ARF} does not appear to play an essential role in the p53 response upon DNA damage (ref 27). Likewise, TBX2 over-expressing cells display a normal cell cycle arrest upon DNA damage (Fig. 3b) and normal induction of the p53 target genes *MDM2* (Fig. 3c) and *p21^{CIP1}*.

*Detailed legend to Figure 3*: ***a***, Growth curves showing that exogenous expression of p16^{INK4a} and p19^{ARF} inhibits proliferation of TBX2 over-expressing MEFs (closed symbols) similar to control MEFs (open symbols). Standard errors were within 10% of the means shown. ***b***, Normal DNA damage induced growth arrest in TBX2 over-expressing, wild-type and *ARF*-/-MEFs, but not in p53-/- MEFs. Results are shown 48 hrs after γ-irradiation. ***c***, Northern blot analysis of MDM2 transcript levels in control (C) and TBX2 (T) over-expressing human TIG3 fibroblasts untreated (-) or treated (+) for 10 hours with 0.2 µg/ml adriamycin.

### TBX2 counteracts Myc- and Ras-mediated induction of p19^{ARF}

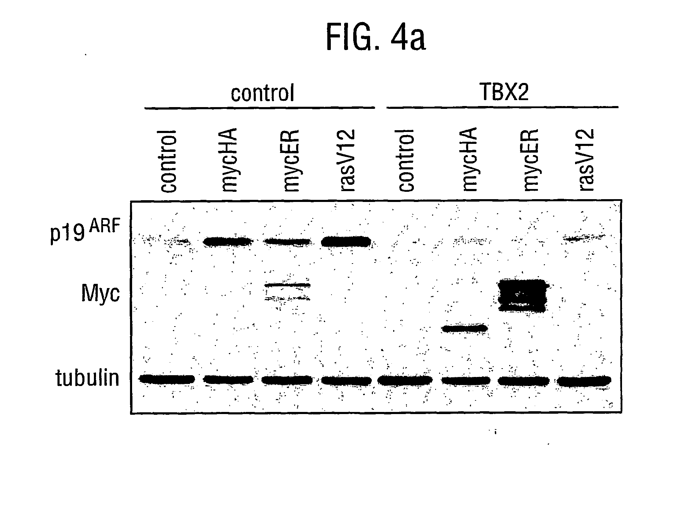

Our previous studies indicated that at least part of Bmi1's oncogenic potential depends on its ability to prevent induction of INK4a/ARF upon aberrant mitogenic signalling (ref. 19). To assess whether TBX2 can mediate similar effects we infected first passage MEFs sequentially with control or TBX2 retroviruses and with control, Myc or RasV12 retroviruses. Western analysis indicated that like Bmi1, TBX2 prevents or attenuates induction of p19^{ARF} by Myc or RasV12 (Fig. 4a).

Over-expression of RasV12 in MEFs elicits a premature senescence arrest (ref 28), which can be noticed by a reduced cell density when these cells are plated and left to proliferate for 2 to 3 weeks. This arrest can be overcome by disruption of the *INK4a*/*ARF* locus, p53 inactivation, or Bmi1 over-expression (refs. 28, 27, 20). When performing such an assay with MEFs co-expressing TBX2 and RasV12, we noticed that TBX2 indeed rescues the impaired proliferative capacity resulting from RasV12 -induced premature senescence entry as judged by their density after 2 weeks in culture. In addition, TBX2/RasV12 co-expressing MEFs acquire a transformed morphology. However in contrast to *ARF-*/*-* or *INK4a*/*ARF-*/*-* MEFs over-expressing RasV12, these cells fail to produce colonies in soft agar, indicating that TBX2 over-expression does not mimic a complete ARF-loss-of-function phenotype. This is in line with the observation that TBX2 over-expressing cells still contain low but detectable levels of p19^{ARF} protein. The observation that TBX2 over-expressing MEFs can proliferate to higher densities than control MEFs in this assay likely reflects the abrogation of senescence in these MEFs.

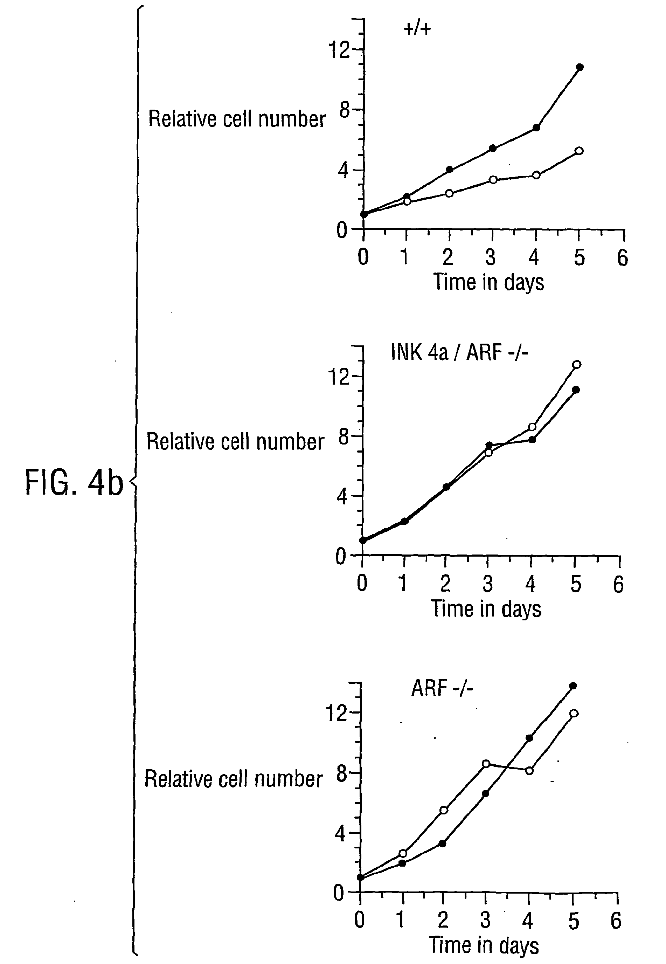

Over-expression of Myc induces an apoptotic response in MEFs that is partially mediated through induction of p19^{ARF} and masks the positive effect of Myc on proliferation (ref. 29). We observed that co-expression of TBX2 partially bypasses this negative effect of Myc, as TBX2 and Myc co-expressing MEFs proliferate faster than MEFs over-expressing only Myc (Fig. 4b). This effect is dependent on ARF, since TBX2 does not further enhance proliferation of Myc over-expressing *INK4a*/*ARF-*/- or *ARF-*/*-* MEFs (Fig. 4b). A similar, although stronger, collaborative effect was observed between Myc and *Bmi1* (ref. 19). However in contrast to MEFs over-expressing both Myc and Bmi1 (ref. 19), TBX2 and Myc co-expressing MEFs do not produce colonies in soft agar.

*Detailed legend to Figure 4: **a**,* Western analysis of p19^{ARF} protein levels of wild-type MEFs infected first with control (C) or TBX2 (T) retroviruses and subsequently with control, MycHA, MycER or RasV12 retroviruses. Although Myc protein levels were equally high, or in this particular experiment even higher, in the TBX2 expressing cells, p19^{ARF} is not efficiently induced in these cells. ***b***, Growth curves showing that TBX2 enhances proliferation of MycHA over-expressing wild-type MEFs, but not of MycHA over-expressing *INK4a*/*ARF-*/*-* or *ARF-*/*-* MEFs (closed symbols: TBX2; open symbols: control). All standard deviations were within 10% of the means shown.

### TBX2 represses the mouse and human ARF promoters

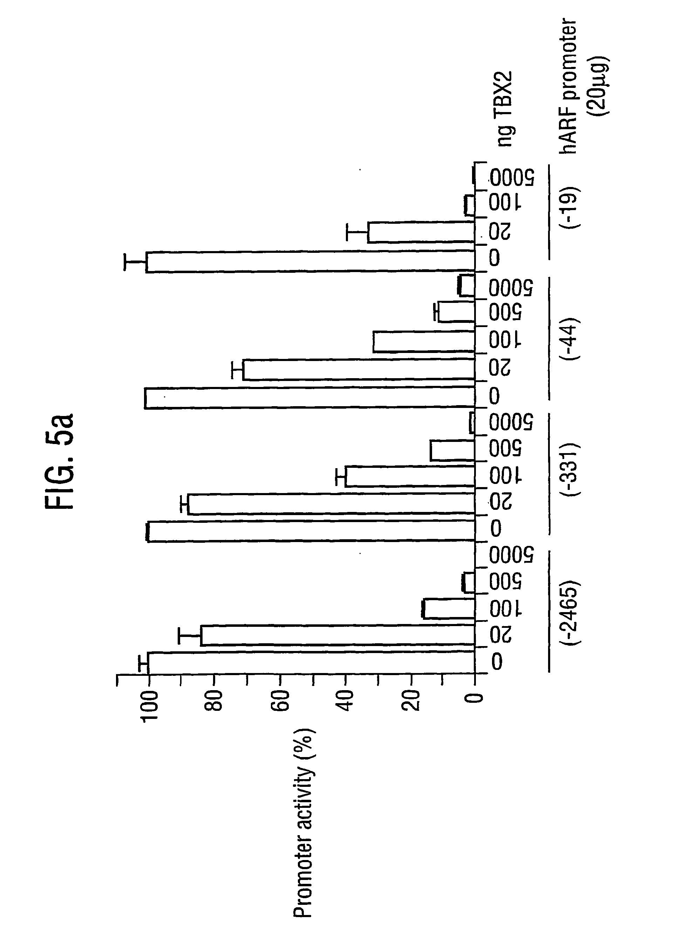

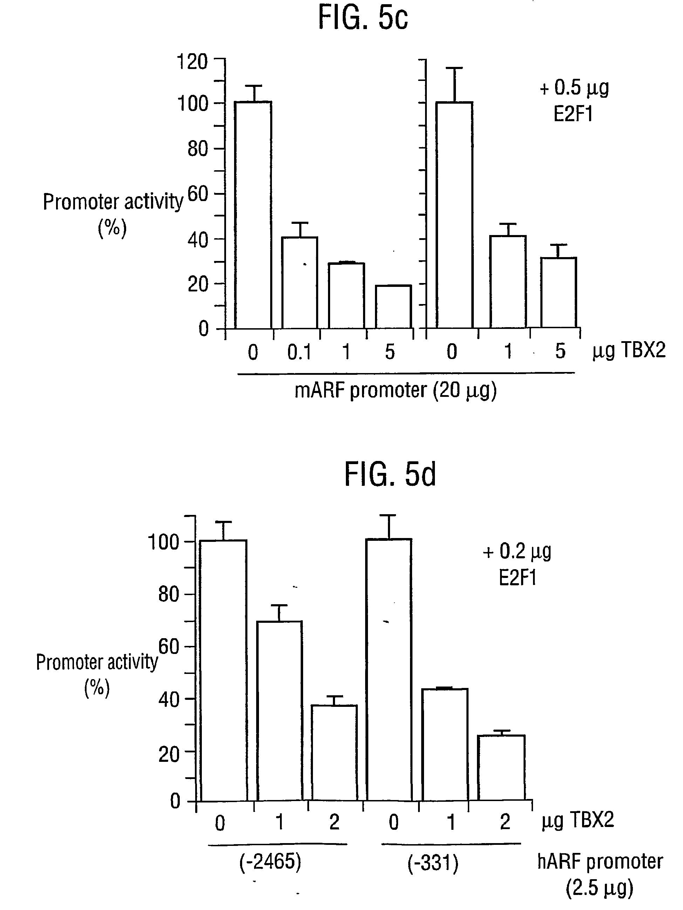

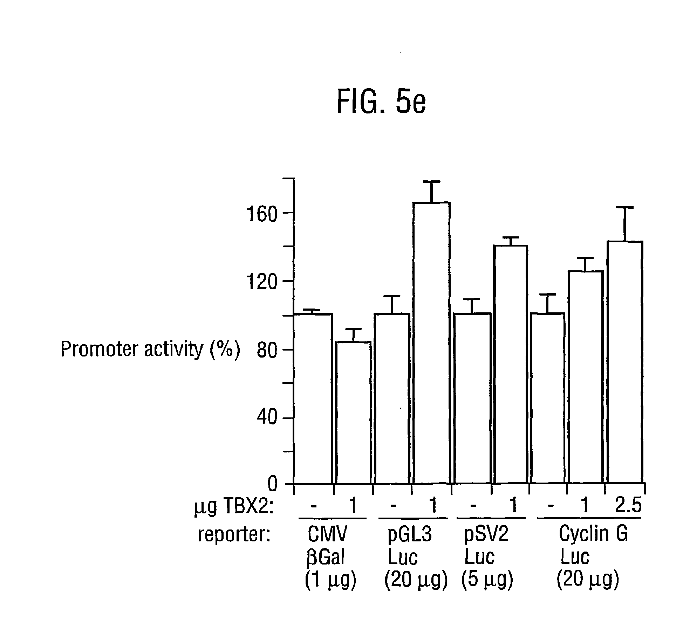

Several regulators of INK4a/ARF expression have been identified, but only a few, including DMP1, p53 and E2F1, were shown to directly affect *INK4a* or *ARF* promoter activity (ref.s 30-32). T-box proteins in general are sequence specific transcriptional activators, however TBX2 and its close homologue TBX3 are thus far exceptions in that they have been shown to also be able to act as transcriptional repressors (refs. 33, 34). Specifically, mouse TBX2 was found to repress the melanocyte-specific tyrosinase-related protein 1 (TRP-1) promoter by binding the consensus T-box binding site GTGTGA, but also GTGTTA and GGGTGA sequences (ref. 33). A GGGTGA sequence is present at position -106 in the human ARF promoter but this site is not conserved in the mouse ARF promoter. To determine whether TBX2 down-regulates p19^{ARF} through direct repression, we performed transient repression assays, using a CAT reporter driven by 5' deletion constructs of the human ARF promoter or a luciferase reporter under control of the 1.7 kb mouse ARF promoter. We used Cos-7 cells for these assays, a cell line that was used before in ARF promoter-studies (ref. 32) and more importantly, appeared not to be hampered by the high levels of TBX2 achieved upon transient transfection. In contrast, we noticed that several other tumour-derived or primary cell lines do not tolerate such high TBX2 levels. The molecular basis for this toxicity is currently under investigation. Interestingly, wild-type TBX2, but not a mutant lacking the recently mapped repression domain (ref. 34), acts as a very strong dose-dependent repressor of both the human and mouse ARF promoter (Fig. 5a-c). That this is a specific effect of TBX2 on the *ARF* promoter and not a global effect on transcription is supported by the observation that TBX2 does not repress the SV40, CMV or Cyclin G promoters in these assays (Fig. 5e). All four human *ARF* promoter deletion constructs tested appear to be strongly repressed by TBX2, suggesting that TBX2 acts through an element located within the region -19 to +54 of the human ARF promoter and not through the putative binding site at position -106. Unfortunately, further mapping of the TBX2 response element was hampered due to loss of promoter activity. In accordance with TBX2 down-regulating p19^{ARF} through direct repression of its promoter, TBX2 does not appear to act indirectly by inducing known negative regulators of the *INK4a*/*ARF* locus, such as Bmi1 (refs 19,20) or Twist (ref. 35), which might also be deduced from their differential effects on the locus.

P19^{ARF} is induced by over-expression of several oncogenes such as *Myc, Ras, E1A* and *E2F1* and as described above, TBX2 can counteract this induction. To analyse whether this occurs at the transcriptional level, we activated both the human and mouse ARF promoter with E2F1 and asked whether TBX2 can inhibit this strong activation. Indeed, we found a dose-dependent inhibition of E2F1 activated *ARF* promoter activity (Fig. 5c+d).

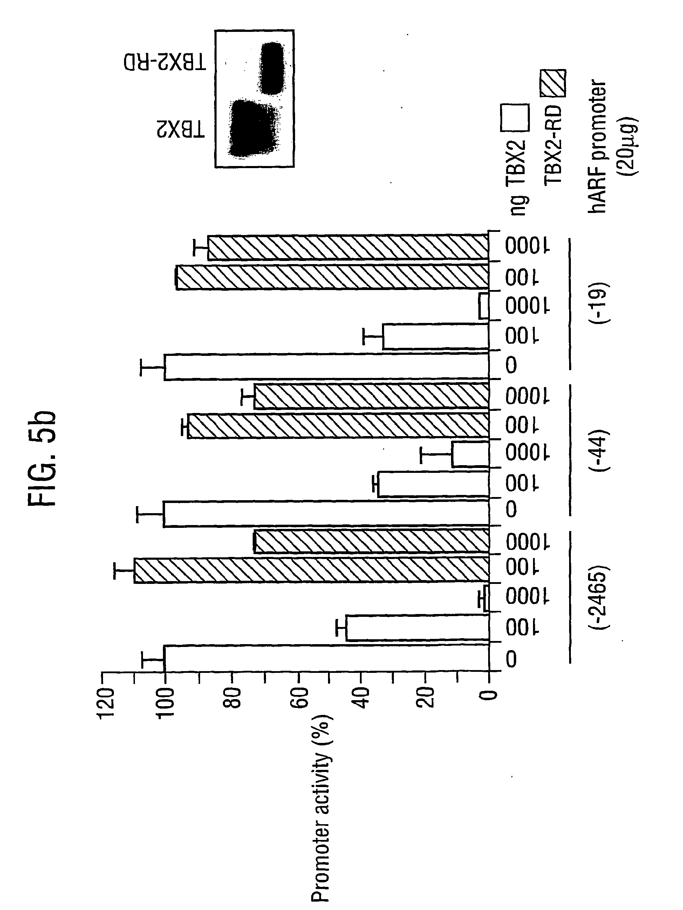

*Detailed legend to Figure 5: **a***, Dose-dependent repression of human *ARF* promoter activity by TBX2. ***b,*** Deletion of the repression domain of TBX2 (TBX2-RD) severely reduces its ability to repress the human ARF promoter. Western analysis shows that the TBX2-RD mutant protein is equally well expressed as the wild-type TBX2. ***c**,* Repression by TBX2 of basal mouse *ARF* promoter activity and E2F1-activated (±25-fold) mouse *ARF* promoter activity. ***d**,* Dose-dependent inhibition by TBX2 of human *ARF* promoter activity after activation (up to 50-fold) with E2F1. ***e**,* TBX2 does not repress the CMV (of CMV-βGal), SV40 (of pGL3promoter-Luc and pSV2-Luc) and rat Cyclin G promoters in COS-7 cells. In all panels the activity of each promoter in the absence of TBX2 is set at 100%.

### ARF promoter analysis.

When looking more closely into the ARF promoter sequences that are still repressed by TBX2 we found out that this region appears to contain a degenerate T-site. An alignment between a consensus T-site region (SEQ ID NO:3) and the corresponding part of the promoter (SEQ ID NO:2) is as follows:

     *  *       *  *
AGTCACACCT-AGGTGTGAAA          (SEQ ID NO:3)
   | |||||  ||||  |||
CTGCTCACCTCTGGTGCCAAA          (SEQ ID NO:2)

Underlined are the sites found in 85% of the *in vitro* site selection experiments with T. Residues marked above with "*" are those found to be important in DNA-binding based on an analysis of the crystal structure of the T-box in complex with the T-site.

We constructed an oligonucleotide based on the ARF T-box like sequence. This oligonucleotide has the sequence:
CTGCTCACCTCTGGTGCCAAA (SEQ ID NO:4).

We performed band shift assays with whole cell extracts of Cos7 cells not over-expressing or over-expressing TBX2 and ³²P-labeled oligonucleotides.

When non-TBX2 over-expressing cell extracts and TBX2 over-expressing cell extracts incubated with the SEQ ID NO:4 oligo are compared, a shift (DNA-protein complex) probably exclusively present in the TBX2-overexpressing extracts was observed. This shift was not affected by pre-immune serum of rabbit but was supershifted both by rabbit polyclonal antibody and mouse monoclonal antibody against TBX2. This strongly indicates that this DNA-protein complex contains TBX2. When we pre-incubated with cold SEQ ID NO:4 oligo we competed out this shift whereas no competition was observed with a mutant oligonucleotide of sequence: CTGCTTGACTCTAAGTCCAAA (SEQ ID NO:5).

Moreover we competed the shift with another oligo of the ARF promoter:

CAGTTAAGGGGGCAGGAGTGGCGCTGCTCACCTCTGGTGCCAAAGGGCGG (SEQ ID
NO:6).

This indicated that the complex formed is specific for the SEQ ID NO:4 and SEQ ID NO:6 oligonucleotides. We also competed this shift with a consensus T-site containing oligonucleotide that has been described to bind TBX2 but, as expected, we competed to a lesser extent with an unrelated, E2F-consensus site containing oligonucleotide. Neither a shift nor a supershift was observed when the same extracts were incubated with the labeled mutant SEQ ID NO:5 oligonucleotide.

### TBX2 amplification in human breast cancer

A gene that not only strongly down-regulates *INK4a*/*ARF* transcription but also prevents induction of INK4a/ARF upon oncogenic hyper-proliferative signalling has great potential to contribute to the development of cancer. Interestingly, *TBX2* resides on human chromosome 17q23 (refs 23,24) a region that is frequently amplified in human breast cancer (refs. 36, 37), in MIII stage meningiomas (ref. 38) and dermatofibrosarcoma protuberans (ref. 39). In breast cancer, amplification of the long arm of chromosome 17 appears to involve three distinct regions: one at 17q21 where the *HER2*/*neu* gene is believed to be the primary target, and two at 17q22-24 (refs 36,37). For the 17q22-24 amplicons it is not known what the primary target genes are, although a good candidate seems to be the serine-threonine kinase *PS6K* (ref. 40). To assess whether TBX2 might be involved in the development of human breast cancer, we first analysed a panel of breast cancer cell lines for amplification of *TBX2*. Southern analysis of human breast cancer cell lines and peripheral blood lymphocytes revealed amplification of *TBX2* in the MCF-7 cell line. *TBX2* amplification was found by northern blot analysis to correlate with significant up-regulation of *TBX2* transcript levels in MCF-7 cells. This cell line has previously been found to also contain amplification of *PS6K* (ref. 40), suggesting that *TBX2* resides on the same amplicon. To substantiate the relevance of *TBX2* amplification in breast tumours, a panel of 177 primary T1/T2 breast carcinomas was screened by Southern analysis. 10 out of 177 tumours showed evident amplification of *TBX2*, with dosage increases from 1.8 to 5.4 (a dosage increase of 2 indicating two extra copies of one *TBX2* allele). None of 5 tumours with clear *TBX2* amplification showed disruption of the *INK4a*/*ARF* locus. In addition, a total of 25 breast carcinomas from patients with a germline mutation in *BRCA1* were analysed by comparative genome hybridization (CGH). Nine tumours showed a high level amplification (fluorescence ratio >> 1.4) on 17q, with 17q22 being the minimal region of overlap. Three of these 9 tumours were available for Southern analysis, 1 showed clear amplification of *TBX2*. Furthermore, CGH analysis of 26 tumours from patients with bilateral breast carcinoma, with unknown BRCA1/2 status, revealed 14 cases of 17q amplification. Six of these were analysed by Southern blot, 2 showed evident *TBX2* amplification. Together these data indicate that the *TBX2* gene is amplified during the genesis of a portion of breast cancers and suggest a potential role for *TBX2* in breast tumour formation.

### Discussion

By exploiting the premature and tight senescence arrest of *Bmi1-*/*-* MEFs we were able to identify the T-box transcription factor TBX2 as a novel negative regulator of the *INK4a*/*ARF* locus, that is amplified in human breast cancer. Although TBX2 significantly down-regulates both p19^{ARF} and p16^{INK4a} transcript levels, long-term immortalization of MEFs by TBX2 appears to be due to strong down-regulation of p19^{ARF}, as TBX2-immortalized MEFs still accumulate p16^{INK4a} protein at levels comparable to those found in senescent cultures. The repression of the *INK4a*/*ARF* locus by TBX2 appears to be sufficient for full immortalization, since TBX2 immortalized MEFs do not display the frequent disruption of the *INK4a*/*ARF* locus or loss of p53 function that is almost invariably associated with spontaneous immortalization of wild-type MEFs. In addition, TBX2 was found to repress transcription from the neighbouring *INK4b* gene. Based on our previous characterization of *Bmil-*/*-* MEFs (ref. 20), the down-regulation of p15^{INK4b} by TBX2 is not likely to be a reason for its ability to immortalize MEFs, although a minor contribution can not be excluded.

The ability of TBX2 to affect the senescence checkpoint is conserved in human cells. However in contrast to MEFs, TBX2 over-expressing human fibroblasts show a delayed entry into senescence rather than a full abrogation. This is not surprising as the latter requires activation of telomere maintenance mechanisms, most commonly through reactivation of the telomerase enzyme (ref. 1), which we found not to be induced by TBX2 as assayed by the telomeric-repeat-amplification protocol (TRAP) assay.

It is believed that activation of the INK4a/ARF checkpoint and the resulting senescence arrest or cell death, acts to prevent potentially cancerous cells from contributing to tumour formation. Consistent with this view are the observations that *ARF-*/*-* and *INK4a*/*ARF-*/*-* mice are highly tumour prone (refs. 27, 28) and that the INK4a/ARF pathway is a frequent target for inactivation in a variety of human cancers (ref. 5). The ability of TBX2 to prevent activation of the ARF checkpoint response upon aberrant mitogenic signalling or increasing population doublings, suggests that TBX2 may have oncogenic potential. This is further supported by the INK4a/ARF-mediated oncogenicity of Bmi1 (ref. 19) and by the inappropriate expression of Twist, another repressor of ARF, in 50% of rhabdomyosarcomas (ref. 35). Like Bmi1, TBX2 alleviates the INK4a/ARF-mediated negative effects of Myc and RasV12 and partially transforms MEFs in combination with RasV12. Interestingly, our studies reveal different threshold effects for p19^{ARF} levels in immortalization and transformation. Reduced levels of p19^{ARF} achieved by TBX2 over-expression clearly allows for efficient immortalization of MEFs and morphological transformation by RasV12 but not for anchorage-independent growth. In contrast, ARF-/- MEFs are not only immortal but also capable of anchorage-independent growth upon over-expression of RasV12 (ref. 27). Clear differences between the biological effects of Bmi1 and TBX2 can also be observed. Firstly, TBX2 induces a change in morphology of MEFs that is clearly distinguishable from that achieved by Bmi1 over-expression. Secondly, in contrast to Bmi1/Myc co-expressing MEFs, TBX2/Myc co-expressing MEFs are unable to grow in soft agar. In addition, Bmi1 but not TBX2 increases the proliferation rate of early passage MEFs. This may indicate an additional role for p16^{INK4a} or other Bmi1 target genes in regulation of proliferation and neoplastic transformation. Alternatively, the positive effects of TBX2, alone or in combination with Ras or Myc, may be counterbalanced in part by the toxicity caused by high levels of TBX2 *in vitro.* In support of this, Bmi1 over-expressing MEF cultures appear to select in favour of the higher expressing cells, whereas cultures infected with TBX2 virus not do so and keep expressing low levels of TBX2. Since TBX2 is a T-box member and a presumed direct target of sonic hedgehog (shh) (ref. 41), one possible explanation is that the toxicity associated with high TBX2 levels results from conflicting signals arising from simultaneous activation of differentiation programs and immortalization.

To our knowledge, our work for the first time associates over-expression of a T-box gene with regulation of the INK4a/ARF checkpoint and the genesis of cancer. This raises the question whether this is a unique feature of TBX2 or whether it is shared by other T-box family members. The observation that immortalization by TBX2 involves its action as a repressor of transcription, together with the differential functions that T-box members appear to have, likely makes it a feature not common to all T-box proteins. Interestingly, TBX2 represses the ARF promoter, however not through binding to DNA sequences that were previously identified as TBX2 interaction sites, but through the -19 to +54 region of the promoter. This region consists almost exclusively of the initiator sequence, suggesting that possibly TBX2 represses the human ARF promoter via initiator interactions. A precedent for such a mechanism is provided by Myc, which activates target genes via binding to E-box sites but represses target genes at least in part via binding to the initiator (ref, 42).

An interesting feature is that TBX2 appears to be directly induced by shh (ref. 41). The *hedgehog (hh)* signalling pathway (ref. 43) is one of the most fundamental signal transduction pathways specifying embryonic development, whose de-regulation not only causes developmental abnormalities but is also clearly implicated in the formation of several human cancers. With TBX2 being a shh target, one novel mechanistic explanation for involvement of hh signalling in cancer might be by crippling the INK4a/ARF checkpoint. In *Drosophila,* one downstream target of hh signalling is *wingless* (ref 44). *Wingless* is a homologue of the mammalian *Wnt* genes (ref. 45) which constitute another major signalling pathway in development that is also implicated in tumourigenesis, including breast cancer development in the mouse (refs. 46, 47). Interestingly, *Wnt* family members have been shown to control several T-box genes, including the *Drosophila* homologue of *TBX2* (refs 48,49). Besides acting on overlapping target genes, *hh* and *Wnt* are also known to act in separate and cooperative pathways during development. The notion that the *c-Myc* gene is a target of the *Wnt* pathway (ref. 50), together with the clear collaboration between c-Myc over-expression and disfunctioning of the INK4a/ARF checkpoint (refs 17-19), suggests one possible way in which deregulated *Wnt* and shh signalling pathways might cooperate in tumourigenesis.

*TBX2* is amplified in a subset of both sporadic and familial human breast cancers, as part of the 17q23 amplicon that has also been shown to contain the *PS6K* gene (ref. 40). Given its multi-functional role, including control of G1-S phase transition, *PS6K* seems a good candidate for being the oncogenic target within this region. However, the ability of TBX2 to prevent triggering of the INK4a/ARF checkpoint upon oncogenic hyper-proliferative signalling and to contribute to immortalization of mouse and human cells makes *TBX2* also an attractive candidate. The degree of amplification of TBX2 in primary breast carcinomas as found by Southern analysis is not very high. A possibility is that this relates to the toxicity we observe in primary cells upon high levels of TBX2 expression. Nevertheless, we have shown that low to moderate over-expression of TBX2, upon viral transduction or transfection of very low amounts of expression plasmid, is very potent in repressing ARF and contributing to immortalization. In this regard it is interesting to note that an additional 4.5% of sporadic breast carcinomas show *TBX2* dosage increases that correspond to one extra copy of the *TBX2* allele. Our findings support the notion that *TBX2* dosage may be diagnostic or prognostic for breast cancer, and provides those of skill in the art with the opportunity to use this in the clinic and in further research.

### Methods

**Retroviral library rescue.** For the primary rescue screen, 10 cm dishes with phoenix producer cells were transfected with 50µg of PMXsubF cDNA library DNA. The construction of the library was as described in Koh et al., (Nucleic Acids Res. (2002) 30(24):e142) Starting 48 hrs post-transfection, viral supernatant was collected 3 times with 12 hrs intervals and used in duplicate for 2-3 consecutive rounds of infection of 10 cm dishes containing 1-2x10⁶ *Bmi1-*/*-* MEFs. Infected MEFs were passaged twice before the appearance of rescued colonies was awaited. Colonies were picked and expanded to allow Southern analysis and Western analysis. For the secondary screen, cells from separate primary rescued colonies were grown to subconfluency in 5 cm dishes and infected with MoMuLV overnight. Following removal of MoMuLV, viral supernatant was collected overnight and used to infect fresh *Bmi1*-/-MEFs. The *TBX2* cDNA was cloned from the rescued cells via a size-selected λ-phage library, as PCR amplification of the insert failed due to the presence of an extremely GC-rich stretch in the 5' part of the *TBX2* cDNA (refs. 23, 24).

**Cell culture, growth curves and retroviral infection**. MEF isolation, cell culture of MEFs and TIG3 fibroblasts, retroviral infections and growth curves were performed as previously described (refs. 19, 20). All experiments were performed multiple times with independently isolated batches of MEFs and were found to be highly reproducible. Retroviruses used were: empty LZRS-iresGFP virus (control), LZRS-Bmil-iresGFP, PMX-TBX2, LZRS-TBX2-iresGFP, pBabePuro-p16^{INK4a}, pBabePuro-p19^{ARF}, pBabePuro-RasV12, LZRS-MycHA-iresGFP and LZRS-MycER-iresGFP. For analysis of lifespan, MEFs and TIG3 cells were propagated on a slightly modified 3T3 schedule by plating every 3.5 days 4x10⁵ cells in a 25 cm² culture flask. To analyse the effect of TBX2 on RasV12 -induced premature senescence, infected cells were plated in 6-wells dishes at a density of 1x10⁵/well and left to grow for 2-3 weeks while refreshing the medium every 3 days. At the end of the assay, plates were fixed and stained with crystal violet.

**Northern and Western blot.** Analysis of p16^{INK4a}, p19^{ARF} and p15^{INF4b} transcript levels was done as described²⁰. For analysis of protein expression, cells were lysed (ref. 20) and equal amounts of protein were separated by SDS-PAGE and blotted onto immobilon-P membranes (Amersham). Western blot analysis was done according to standard methods using enhanced chemiluminescence (Amersham). Primary antibodies used were M-156 for mouse p16^{INK4a} (Santa Cruz) and R562 for mouse p19^{ARF} (abcam). The antibody used for detection of TBX2 protein was generated as polyclonal sera in both rabbit and mice.

**Analysis of DNA damage response.** MEFs were exposed to 8 Gy γ-irradiation using a ¹³⁷Cs γ-beam at a rate of 0.75 Gy/min. After 48 hrs, treated and untreated cells were incubated with 10 µM BrdU for 2 hrs, trypsinized and fixed with 70% ethanol. Fixed cells were incubated for 30 min. at 37°C with 0.5 mg/ml RNase, washed with PBS and incubated for 20 min. at RT in 5 M HCl/0.5 % Triton-X100. Samples were incubated with anti-BrdU monoclonal antibody (1:40, Dako) in PBS containing 0.5 % Tween-20 and 1 % BSA and subsequently with FITC-conjugated anti-mouse IgG (1:20, Monosan). Cells were stained with 20µg/ml propidium iodide and analysed by flow-cytometry. For analysis of p21^{cip1} and MDM2 induction upon DNA damage, control and TBX2 over-expressing human TIG3 fibroblasts were untreated or treated for 10 hrs with 0.2 µg/ml adriamycin.

**Repression assays.** For analysis of TBX2-mediated repression of the ARF promoter, Cos-7 cells were transfected in 10 cm dishes at approximately 60% confluency by calcium-phosphate precipitation with (-2465), (-331), (-44) and (-19) human *ARF-*promoter-CAT reporter plasmids (ref. 32) or with a (-1680) mouse *ARF*-promoter-Luciferase reporter plasmid, in combination with 0-5000 ng of pCDNA3.1 expression plasmid (Invitrogen) containing the complete cDNA of *TBX2*, or a mutant cDNA lacking the repression domain (TBX2-RD). TBX2-RD was generated by removing an internal *BstEII* fragment covering the repression domain (ref. 34). For analysis of TBX2-mediated inhibition of E2F1 activated *ARF* promoter activity, CMV-E2F1 expression plasmid was co-transfected. All transfections contained equal amounts of pCDNA3.1 and included 1 µg pSVβgal plasmid (Promega) as an internal transfection control. Additional negative controls were CMV-βgal (Clontech), pGL3promoter-Luc, pSV2-Luc (Promega) and rat Cyclin G-promoter-Luc (kindly provided by M.Oren). Analysis of CAT (chloramphenicol-acetyl-transferase), luciferase and β-galactosidase activity 48 hours after transfection was done according to standard methods.

**CGH and analysis of *TBX2* amplification and over-expression**. CGH was performed on DNA isolated from formalin fixed paraffin embedded tissue as described (ref. 37). 400 ng biotin-labelled tumour DNA was hybridized with 400 ng rhodamin-labelled reference DNA in the presence of 25 µg cot1 DNA. Image analysis was performed using Vysis software (Vysis, USA). For detection of *TBX2* amplification, Southern blots containing 15 µg of *EcoRI*-digested genomic DNA were hybridized with a ³²P-labelled EcoRl fragment derived from the 3' (primary tumours) or 5' (cell lines) portion of the human *TBX2* cDNA. The same 3' probe was used on Northern blots for detection of *TBX2* over-expression.

### References

1. Wynford-Thomas, D. Cellular senescence and cancer. *J. Pathol*. **187,** 100-111 (1999).
2. Levine, A.J. p53, the cellular gatekeeper for growth and division. *Cell* **88,** 323-333 (1997).
3. Serrano, M., Hannon, G.J. & Beach , D. A new regulatory motif in cell -cycle control causing specific inhibition of cyclin D/CDK4. *Nature* **366**, 704-707 (1993).
4. Alcorta, D.A. *et al*. Involvement of the cyclin-dependent kinase inhibitor p16(INK4a) in replicative senescence of normal human fibroblasts. *Proc. Natl. Acad. Sci. USA* **93,** 13742-13747 (1996).
5. Ruas, M. & Peters, G. The p16INK4a/CDKN2A tumor suppressor and its relatives. *Biochim. Biophys. Acta* **1378,** F115-F177 (1998).
6. Palmero, I. *et al*. Accumulation of p16^{INK4a} in mouse fibroblasts as a function of replicative senescence and not of retinoblastoma gene status. *Oncogene* **15**, 495-503 (1997).
7. Sherr, C.J. Tumor surveillance via the ARF-p53 pathway. *Genes Dev.* **12**, 2984-2991 (1998).
8. Sharpless, N.E. & DePinho, R.A. The *INK4A*/*ARF* locus and its two gene products. *Curr. Opin. Genet. Dev.* **9,** 22-30 (1999).
9. Quelle, D.E., Zindy, F., Ashmun, R.A. & Sherr, C.J. Alternative reading frames of the INK4a tumor suppressor gene encode two unrelated proteins capable of inducing cell cycle arrest. Cell **83**, 993-1000 (1995).
10. Kamijo, T. *et al*. Functional and physical interactions of the ARF tumor suppressor with p53 and Mdm2. *Proc. Natl. Acad. Sci. USA* **95,** 8292-8297 (1998).
11. Pomerantz, J. *et al*. The Ink4a tumor suppressor gene product p19^{ARF}, interacts with MDM2 and neutralizes MDM2' s inhibition of p53. *Cell* **92**, 713-723 (1998).
12. Stott, F.J. *et al*. The alternative product from the human CDKN2A locus, p14^{ARF}, participates in a regulatory feedback loop with p53 and MDM2. *EMBO* J. **17**, 5001-5014 (1998).
13. Zhang, Y., Xiong, Y. & Yarbrough, W.G. ARF promotes MDM2 degradation and stabilizes p53: ARF-INK4a locus deletion impairs both the Rb and p53 tumor suppression pathways. *Cell* **92**, 725-734 (1998).
14. Weber, J.D., Taylor, L.J., Roussel, M.F., Sherr, C.J. & Bar-Sagi, D. Nucleolar ARF sequesters Mdm2 and activates p53. *Nature Cell Biol.* **1**, 20-26 (1999).
15. Tao, W. & Levine, A.J. P19^{ARF} stabilizes p53 by blocking nucleo-plasmic shuttling of Mdm2. *Proc. Natl. Acad. Sci. USA* **96,** 6937-6941 (1999).
16. Honda, R. & Yasuda, Y. Association of p19^{ARF} with Mdm2 inhibits ubiquitin ligase activity of MDM2 for tumor suppressor p53. *EMBO J.* **18**, 22-27 (1999).
17. Eischen C.M., Weber, J.D., Roussel, M.F., Sherr, C.J. & Cleveland, J.L. Disruption of the ARF-Mdm2-p53 tumor suppressor pathway in Myc-induced lymphomagenesis. *Genes Dev.* **13,** 2658-2669 (1999).
18. Schmitt, C.A., McCurrach, M.E., de Stanchina, E., Wallace-Brodeur, R.R. & Lowe, S.W. INK4a/ARF mutations accelerate lymphomagenesis and promote chemoresistance by disabling p53. *Genes Dev.* **13,** 2670-2677 (1999).
19. Jacobs, J.J.L. *et al*. Bmi1 collaborates with c-Myc in tumorigenesis by inhibiting c-Myc-induced apoptosis via INK4a/ARF. *Genes Dev.* **13**, 2678-2690 (1999).
20. Jacobs, J.J.L., Kieboom, K., Marino, S., DePinho, R.A. & van Lohuizen, M. The oncogene and Polycomb-group gene *Bmi1* regulates cell proliferation and senescence through the INK4a locus. *Nature* **397**, 164-168 (1999).
21. Haupt, Y., Bath, M.L., Harris, A.W. & Adams, J.M. Bmi1 transgene induces lymphomas and collaborates with myc in tumorigenesis. *Oncogene* **8**, 3161-3164 (1993).
22. Alkema, M.J., Jacobs, H., van Lohuizen, M. & Berns, A. Perturbation of B and T cell development and predisposition to lymphomagenesis in Eµ-*Bmi1* transgenic mice require the Bmi1 RING finger. *Oncogene* **15**, 899-910 (1997).
23. Campbell, C., Goodrich, K., Casey, G. & Beatty, B. Cloning and mapping of a human gene (*TBX2*) sharing a highly conserved protein motif with the Drosophila omb gene. *Genomics* **28,** 255-260 (1995).
24. Law, D.J., Gebuhr, T., Garvey, N., Agulnik, S.I. & Silver, L.M. Identification, characterization, and localization to chromosome 17q21-22 of the human *TBX2* homolog, member of a conserved developmental gene family. *Mamm. Genome* **6,** 793-797 (1995).
25. Papaioannou, V.E. & Silver, L.M. The T-box gene family. *Bioessays* **20,** 9-19 (1998).
26. Quelle, D.E. *et al*. Cloning and characterization of murine p16INK4a and p15INK4b genes. *Oncogene* **11,** 635-645 (1995).
27. Kamijo, T.F. *et al*. Tumor suppression at the mouse INK4a locus mediated by the alternative reading frame product p19^{ARF}. *Cell* **91,** 649-659 (1997).
28. Serrano, M. *et al*. Role of the INK4a locus in tumor suppression and cell mortality. *Cell* **85,** 27-37 (1996).
29. Zindy, F. *et al*. Myc signaling via the ARF tumor suppressor regulates p53-dependent apoptosis and immortalization. *Genes Dev.* **12**, 2424-2433 (1998).
30. Inoue, K., Roussel, M.F. & Sherr, C.J. Induction of ARF tumor suppressor gene expression and cell cycle arrest by transcription factor DMP1. *Proc. Natl. Acad. Sci. USA* **96,** 3993-3998 (1999).
31. Bates, S. *et al*. p14^{ARF} links the tumour suppressors RB and p53. *Nature* **395,** 124-125 (1998).
32. Robertson, K.D. & Jones, P.A. The human ARF cell cycle regulatory gene promoter is a CpG island which can be silenced by DNA methylation and down-regulated by wild-type p53. *Mol. Cell. Biol.* **18,** 6457-6473 (1998).
33. Carreira, S., Dexter, T.J., Yavuzer, U., Easty, D.J. & Goding, C.R. Brachyury-related transcription factor Tbx2 and repression of the melanocyte-specific TRP-1 promoter. *Mol. Cell. Biol.* **18,** 5099-5108 (1998).
34. He, M.I., Wen, L., Campbell, C.E., Wu, J.Y. & Rao, Y. Transcription repression by Xenopus ET and its human ortholog TBX3, a gene involved in ulnar-mammary syndrome. *Proc. Natl. Acad. Sci. USA* **96,** 10212-10217 (1999).
35. Maestro, R. *et al*. Twist is a potential oncogene that inhibits apoptosis. *Genes Dev.* **13,** 2207-2217 (1999).
36. Courjal, F. & Theillet, C. Comparative genomic hybridization analysis of breast tumors with predetermined profiles of DNA amplification. *Cancer Res.* **57,** 4368-4377 (1997).
37. Barlund, M. *et al*. Increased copy number at 17q22-24 by CGH in breast cancer is due to high-level amplification of two separate regions. *Genes Chromosomes Cancer* **20,** 372-376 (1997).
38. Weber, R.G. *et al*. Analysis of genomic alterations in benign, atypical, and anaplastic meningiomas: toward a genetic model of meningioma progression. *Proc. Natl. Acad. Sci. USA* **94,** 14719-14724 (1997).
39. Pedeutour, F. *et al*. Ring 22 chromosomes in dermatofibrosarcoma protuberans are low-level amplifiers of chromosome 17 and 22 sequences. *Cancer Res.* **55,** 2400-2403 (1995).
40. Couch, F.J. *et al*. Localization of PS6K to chromosomal region 17q23 and determination of its amplification in breast cancer. *Cancer Res.* **59,** 1408-1411 (1999).
41. Gibson-Brown, J.J., Agulnik, S.I., Silver, L.M., Niswander, L. & Papaioannou, V.E. Involvement of T-box genes Tbx2-Tbx5 in vertebrate limb specification and development. *Development* **125,** 2499-2509 (1998).
42. Li, L.-H., Nerlov, C., Prendergast, G., MacGregor, D. & Ziff, E.B. c-Myc represses transcription in vivo by a novel mechanism dependent on the initiator element and Myc box II. *EMBO* J. **13,** 4070-4079 (1994).
43. Wicking, C., Smyth, I. & Bale, A. The hedgehog signaling pathway in tumorigenesis and development. *Oncogene* **18,** 7844-7851 (1999).
44. Ingham, P.W. Localized hedgehog activity controls spatial limits of wingless transcription in the Drosophila embryo. *Nature* **366,** 560-562 (1993).
45. Miller, J.R., Hocking, A.M., Brown, J.D. & Moon, R.T. Mechanism and function of signal transduction by the Wnt/β - catenin and Wnt/Ca²⁺ pathways. *Oncogene* **18,** 7860-7872 (1999).
46. Nusse, R. & Varmus, H.E. Many tumors induced by the mouse mammary tumor virus contain a provirus integrated in the same region of the host genome. Cell **31,** 99-109 (1982).
47. Roose, J. *et al*. Synergy between tumor suppressor APC and the β-catenin-Tcf4 target Tcf1. *Science* **285,** 1923-1926 (1999).
48. Grimm, S. & Pflugfelder, G.O. Control of the gene *optomotor-blind* in *Drosophila* wing development by *decapentaplegic* and *wingless. Science* **271,** 1601-1604 (1996).
49. Yamaguchi, T.P., Takada, S., Yoshikawa, Y., Wu, N. & McMahon, A.P. T (Brachyury) is a direct target of Wnt3a during paraxial mesoderm specification. *Genes Dev.* **13**, 3185-3190 (1999).
50. He, T.-C. *et al*. Identification of c-MYC as a target of the APC pathway. *Science* **281,** 1509-1512 (1998).

### SEQUENCE LISTING:

SEQ ID NO:1
TCTGCAGTTA AGGGGGCAGG AGTGGCGCTG CTCACCTCTG GTGCCAAAGG
GCGGCGCAGC GGCTGCCGAG CTC

SEQ ID NO:2
   CTGCTCACCT CTGGTGCCAA A
SEQ ID NO:3
   AGTCACACCT AGGTGTGAAA
SEQ ID NO:4
   CTGCTCACCT CTGGTGCCAA A
SEQ ID NO:5
   CTGCTTGACT CTAAGTCCAA A
SEQ ID NO:6
   CAGTTAAGGG GGCAGGAGTG GCGCTGCTCA CCTCTGGTGC CAAAGGGCGG

## Claims

1. An assay for a modulator of cell cycle progression, which comprises:
a) bringing into contact a TBX2 protein, an ARF promoter TBX-2 binding region and a putative inhibitor compound under conditions where the TBX2 protein and the binding region, in the absence of inhibitor, are capable of forming a complex; and
b) measuring the degree of inhibition of binding or transcriptional activation caused by said inhibitor compound.

2. An assay according to claim 1 wherein said binding region is operably linked to a reporter gene.

3. A method for the diagnosis or prognosis of breast cancer which method comprises determining the amount of TBX2 nucleic acid or protein in a cell which cell is, or is suspected to be, a breast tumour cell or metastatic cell from a breast tumour.

4. A method according to claim 3 wherein the copy number of genomic TBX2 is determined.

5. A method according to claim 3 wherein the amount of TBX2 is determined by the use of an immunoassay to determine the amount of TBX2 protein.

## Patentansprüche

1. Test für einen Modulator der Zellzyklusprogression, umfassend:
a) das In-Kontakt-Bringen eines TBX2-Proteins, einer ARF-Promotor-TBX-2-Bindungsregion und einer vermutlichen Inhibitorverbindung unter Bedingungen, unter denen das TBX2-Protein und die Bindungsregion in Abwesenheit eines Inhibitors zur Bildung eines Komplexes in der Lage sind; und
b) das Messen des Grads der Hemmung von Bindung oder Transkriptionsaktivierung aufgrund dieser Inhibitorverbindung.

2. Test nach Anspruch 1, worin die Bindungsregion operabel an ein Reportergen gebunden ist.

3. Verfahren zur Diagnose oder Prognose von Brustkrebs, wobei das Verfahren das Bestimmen der Menge an TBX2-Nucleinsäure oder -Protein in einer Zelle umfasst, wobei die Zelle eine Brusttumorzelle oder Metastasenzelle von einem Brusttumor ist oder solch ein Verdacht besteht.

4. Verfahren nach Anspruch 3, worin die Kopienzahl von genomischem TBX2 bestimmt wird.

5. Verfahren nach Anspruch 3, worin die Menge an TBX2 durch den Einsatz eines Immuntests zur Bestimmung der Menge an TBX2-Protein bestimmt wird.

## Revendications

1. Essai pour un modulateur de la progression du cycle cellulaire, qui comprend :
a) la mise en contact d'une protéine TBX2, d'une région de liaison de TBX2 à un promoteur ARF et d'un composé inhibiteur putatif dans des conditions où la protéine TBX2 et la région de liaison, en l'absence de l'inhibiteur, peuvent former un complexe ; et
b) la mesure du degré d'inhibition de la liaison ou de l'activation de transcription provoquée par ledit composé inhibiteur.

2. Essai selon la revendication 1 où ladite région de liaison est opérativement enchaînée à un gène reporteur.

3. Méthode pour le diagnostic ou le pronostic d'un cancer du sein, laquelle méthode consiste à déterminer la quantité d'acide nucléique de TBX2 ou de la protéine dans une cellule, laquelle cellule est ou est suspectée d'être une cellule de tumeur du sein ou une cellule métastatique provenant d'une tumeur du sein.

4. Méthode selon la revendication 3, où le nombre de copies de TBX2 génomique est déterminé.

5. Méthode selon la revendication 3, où la quantité de TBX2 est déterminée par l'utilisation d'un immunoessai pour déterminer la quantité de la protéine TBX2.
